# EUROPEAN PATENT SPECIFICATION

(11) **EP 1 789 531 B1**
(45) Date of publication and mention of the grant of the patent: **13.10.2010**
(21) Application number: 05765902.1
(22) Date of filing: 30.06.2005
(51) Int. Cl.: C12N 1/20, A23L 1/054, A61K 35/66

(54) **LACTOBACILLUS RHAMNOSUS WITH BODY-FAT REDUCING ACTIVITY AND THE FOODS CONTAINING THEM**
LACTOBACILLUS RHAMNOSUS MIT KÖRPERFETT REDUZIERENDER AKTIVITÄT SOWIE DIESE ENTHALTENDE LEBENSMITTEL
LACTOBACILLUS RHAMNOSUS A ACTIVITE DE REDUCTION DE GRAISSE CORPORELLE ET ALIMENTS LES CONTENANT

(30) Priority: 16.08.2004 KR 2004064474
(43) Date of publication of application: 30.05.2007
(73) Proprietor: PL Bio Co., Ltd., Gwanak-gu Seoul 151-010 (KR); CJ Cheiljedang Corp., Jung-gu Seoul 100-802 (KR)
(72) Inventor: LEE, Yeon-Hee, Dogok-dong, Gangnam-gu, Seoul 135-270 (KR); PAEK, Kyung-Soo, Dogok-dong, Gangnam-gu, Seoul 135-270 (KR); KOH, Jee-Hoon, Seodaemun-gu, Seoul 120-762 (KR); KIM, Tae-JIn, Guro-gu, Seoul 152-752 (KR); PARK, Bum-Suk, Guro-gu, Seoul 152-773 (KR); SOHN, Kenny, Yongsin-si, Gyeonggi-do 448-515 (KR)
(74) Representative: Portal, Frédéric
(86) International application number: PCT/KR2005/002066
(87) International publication number: WO 2006/019222

(56) References cited:
- EP-A- 1 034 788
- EP-A- 1 174 416
- EP-A1- 1 500 706
- WO-A-01/36653
- WO-A-03/087344
- KR-A- 2004 064 157
- US-A- 6 060 304
- LARSEN THOMAS M ET AL: "Efficacy and safety of dietary supplements containing CLA for the treatment of obesity: Evidence from animal and human studies." JOURNAL OF LIPID RESEARCH, vol. 44, no. 12, December 2003 (2003-12), pages 2234-2241, XP002467955 ISSN: 0022-2275
- LEE S.O. ET AL: 'Bioconversion of linoleic acid into conjugated Linoleic acid during fermentation and by washed cells of lactobacillus reuteri' BIOTECHNOL. LETTERS vol. 25, no. 12, 2003, pages 935 - 938, XP008092146
- KISHINO S. ET AL: 'Structural analysis of conjugated linoleic acid produced by Lactobacillus plantarum, and factors affecting isomer production' BIOSCI. BIOTECHNOL. BIOCHEM. vol. 67, no. 1, 2003, pages 179 - 182, XP008092144

## Description

### [Technical Field]

The present invention relates to a *Lactobacillus rhamnosus* strain with body-fat reducing activity and foods containing it. The present invention provides a *Lactobacillus* strains with body-fat reducing activity, wherein said strain is *Lactobacillus rhamnosus* Strain PL60 KCCM-10654P. The present invention also provides live or dried Lactobacillus strains of the present invention converting LA into CLA with a body-fat reducing effect, and body-fat reducing functional foods and food additives containing them. In addition, the present invention provides body-fat reducing functional foods and beverages using the Lactobacillus strain of the invention with a body-fat reducing effect as a starter strain or additive. Furthermore, the present invention provides a medicament containing the Lactobacillus strains of the present invention.

In addition, the present invention provides a mass-producing process of CLA using the strains of the present invention.

### [Background Art]

In modem societies, obesity is a disease with lower perfect cure proportion than cancer and increases a death rate as well as various adult diseases resulting 25 from it. It has brought about severe problems enough to make public "war on obesity" in U.S.A. Many materials have been asserted to be a material effective in preventing and treating obesity, but till now only pyruvic acid and conjugated linoleic acid (CLA) have been proved to be efficacious according to a scientific basis (Lenz TL, Hamilton WR. Supplemental products used for weight loss. 2004. J Am Pharm Assoc (Wash DC) 44:59-67). It is suggested that a body-fat reducing mechanism is a reduction of adipose-cell number, a reduction of adipose-cell size, an ingestion reduction of energy and food, a production reduction of fat, an increase of energy consumption, lipolytic activity, an increase of lipid oxidation or like by inducing apoptosis of adipose cells(Chardigny JM, Hasselwander O, Genty M, Kraemer K, Ptock A, Sebedio JL. 2003, Effect of conjugated FA on feed intake, body composition, and liver FA in mice. Lipids. 38(9):895-902).

CLA(c9t11-octadecadienoic acid, t10c12-octadecadienoic acid) is formed through an isomerization of linoleic acid(LA, C18:2 cis9cis12). It has been known that CLA has an antioxidative effect, a cholesterol lowering effect, a growth promoting effect, and an anticancer effect according to the location of double bonds. Recently, it has bee known that CLA has body plasma lipids, a body-fat reducing effect, or like. It has been reported that CLA may be found in animal meats, fermented milk or like. Animal experiments and clinical trials have already proved that especially c9,t11-CLA of CLA isomers has a body-fat reducing effect. Most ideally, c9t11-CLA and t10c12-CLA are most preferably produced in the same quantity.

*Butyrivibrio fibriosolvents* is the first found anaerobic microorganism that produces CLA, which is isolated from ruminants like a cow. It produces trans-11-octadecenoic acid through 2 steps upon the biohydrogenation of LA. cis-9, trans-11-Octadecadienoic acid is produced by the action of linoleic acid isomerases, prior to hydrogenating the generated conjugated acid to produce trans-11-octadecenoic acid.

According to the recent Norway study in 2004(Gaullier JM, Halse J, Hoye K, Kristiansen K, Fagertun H, Vik H, Gudmundsen O. 2004. Conjugated linoleic acid supplementation for 1 y reduces body fat mass in healthy overweight humans. Am J Clin Nutr. 79(6):1118-1125), CLA caused a weight loss of 4-10% without side effects when administered to 180 overweight people for one year.

The present invention selected and identified a Korean-type *Lactobacillus* strain with a body-fat reducing effect that overproduced t10c12-CLA, confirmed characteristics of a probiotic, such as intestinal adaptation or like, in the strain, and found out conditions that the strain could maximally produce CLA and *Lactobacillus* strains with a body fat reducing effect by carrying out an animal experiment to confirm weight loss.

### [Disclosure]

### [Technical Problem]

Therefore, the present invention has been made in view of the above problems, and it is an object of the present invention to provide a *Lactobacillus* strain that produces CLA has a body-fat reducing effect, good adhesion to the intestines, and strong tolerance to both acid and bile, wherein said strain is *Lactobacillus rhamnosus* Strain PL6O that was deposited as KCCM-10654P to Korean Culture Center of Microorganisms on May 9^{th}, 2005.

Another object of the present invention is to provide *a Lactobacillus* strain that reduces body fat, wherein said strain is *Lactobacillus rhamnosus* Strain PL60 KCCM-10654P, as a medicament and for use in preventing or treating obesity-related diseases.

Still another object of the present invention is to provide a mass-producing process of CLA with a body-fat reducing effect using *Lactobacillus rhamnosus* strain PL6O KCCM-10654P.

The probiotic *Lactobacillus rhamnosus* strain PL6O KCCM-10654P of the invention doesn't transfer an antibiotic resistance and is harmless.

*Lactobacillus* strains can be prepared in various compositions, preferably these compositions are compositional forms, such as capsules, tablets, powder etc and convenient forms capable of being added to various foods.

These formulations can be prepared using pharmaceutically acceptable carriers, excipients, solvent or supplements by the known methods. These method and ingredients have been well known, and are in detail disclosed in standard texts and manuals, for example a publication(Remington. 1995. The Science and Practice of Pharmacy. Mack Publishing Co. Easton, PA 18042, USA).

Digestive Foods containing *Lactobacillus* strains may be prepared by the well-known method in the art.

Foods and beverages with a body-fat reducing effect may be prepared by the well-known method in the art using the strain as a starter strain or additive of fermented foods containing fermented milk products.

Fermented foods with a maximum body-fat reducing effect can be produced using conditions suggested herein.

Hereinafter, the present invention is explained in more detail.

### [Technical Solution]

In accordance with an aspect of the present invention, the above and other objects can be accomplished by the provision of body-fat reducing functional foods, comprising Lactobacillus rhamnosus Strain PL60 KCCN-10654P.

In accordance with another aspect of the present invention, there is provided *Lactobacillus rhamnosus* Strain PL60 KCCM-10654P capable of reducing body fat.

In accordance with another aspect of the present invention, there are provided body-fat reducing functional foods containing *Lactobacillus rhamnosus* Strain PL60 KCCM-10654P in an amount of 1 × 10⁶ - 1 × 10¹¹ CFU/g in order to prevent and treat adult diseases using a body-fat reducing effect.

In accordance with another aspect of the present invention, there are provided food and beverage additives containing *Lactobacillus rhamnosus* Strain PL60 KCCM-10654P.

In accordance with another aspect of the present invention, there are provided conditions capable of obtaining a maximum body-fat reducing effect in fermented foods using *Lactobacillus rhamnosus* Strain PL60 KCCM-10654P.

Hereinafter, the present invention will be described in more detail by reference to examples of preferred embodiments of the present invention which, however, are not to be construed as limiting the present invention in any way.

### [Advantageous Effects]

*Lactobacillus rhamnosus* Strain PL60 of the present invention has a body-fat reducing effect to be capable of preventing or treating diseases resulting from obesity. In addition, dried *Lactobacillus rhamnosus* Strain PL60 and *Lactobacillus rhamnosus* Strain PL60 cultural filtrates, dried cultural filtrates may be used as additives of various foods and beverages to be useful in preventing and treating body fat, hence can be used in preventing and treating all obesity-related diseases. Furthermore, fermented foods using said *Lactobacillus rhamnosus* Strain PL60 of the present invention could prevent and treat obesity by a body-fat reducing effect.

In addition, according to the present invention *Lactobacillus rhamnosus* Strain PL60 must be primary-cultured in a medium containing LA in order to produce maximum CLA. LA content is 100-1000ppm, Tween-80 content is 1-0.1%, and carbohydrate is preferably fructose, sucrose, and lactose, most preferably fructose, so that fermented foods using *Lactobacillus rhamnosus* Strain PL60 have a maximum body fat reducing effect.

### [Description of the Drawings]

The above and other objects, features and other advantages of the present invention will be more clearly understood from the following detailed description taken in conjunction with the accompanying drawings, in which:
Fig. 1 is a gas chromatogram identifying CLA generated by *Lactobacillus rhamnosus* Strain PL60.
Fig. 2 is a micrograph of *Lactobacillus rhamnosus* Strain PL60.
Fig. 3 shows the 16S rRNA sequence of *Lactobacillus rhamnosus* Strain PL60.
Fig. 4 is band patterns identifying *Lactobacillus rhamnosus* Strain PL60 using a multiplex PCR.
Fig. 5 shows the experimental results for an adaptation of *Lactobacillus rhamnosus* Strain PL60 to Caco-2 cells.
Fig. 6a and 6b show the experimental results for an adhesion of *Lactobacillus rhamnosus* Strain PL60 to the human intestines.
Fig. 7 is band patterns illustrating PCR results of an isolated colony after orally administrating *Lactobacillus rhamnosus* Strain PL60 to people.
Fig. 8 shows the changes of the body weight of rats that took *Lactobacillus rhamnosus* Strain PL60.
Fig. 9 is a graph comparing the body weight of rats of each group after administrating *Lactobacillus rhamnosus* Strain PL60 on the 10^{th} week.

### [Best Mode]

### Example 1: Screening of Lactobacillus Strains Capable of Producing Conjugated Linoleic Acid (hereafter, referred to CLA)

In order to select CLA-producing strains, *Lactobacillus* strains that grew in a medium containing LA, a substrate of CLA, were screened. And then, it was confirmed whether they expressed an isomerase enzyme, an enzyme involved in producing CLA.

### <Materials and Method>

First, *Lactobacillus* strains that grew in a medium containing linoleic acid(LA) were selected, of which CLA-producing *Lactobacillus* strains were screened. For this, CLA-producing strains may easily be screened from a large quantity of *Lactobacillus* strains by using an isomerase assay(Ogawa J, Matsumura K, Kishino S, Omura Y, and Shimizu S. 2001. Conjugated linoleic acid accumulation via 10-Hydroxy-12-octadecaenoic acid during microaerobic transformation of linoleic acid by Lactobacillus acidophilus. Appl. Envir. Microbiol. 67:1246-1252). First, *Lactobacillus* strains that grew in a MRS medium containing 0.1% LA were primarily selected. And then, these *Lactobacillus* strains were twice subcultured in a MRS broth and cultured in a MRS broth containing 0.1 % LA 10mL for 2 days. The medium of 5mL was centrifuged at 8000rpm for 10 minutes to collect cells, prior to washing the cells with a 0.1M potassium phosphate buffer solution(pH 7.0). Again, thereto a 0.1M potassium phosphate buffer solution(pH 7.0) 1.0mL was added, followed by breaking and centrifuging the admixture every 3 minutes in a cold state using an ultrasonic breaker to obtain a crude enzyme solution. The crude enzyme solution was added to a substrate solution(LA 0.1mL, 0.1M potassium phosphate buffer 2.7mL, and 1,3-propanediol 0.2mL) to measure an absorbance at 233nm.

### <Results and Discussion>

CLA-producing *Lactobacillus* strains were screened out of more than 200 *Lactobacillus* strains using an isomerase assay.

### Experimental Example 1: Identification of CLA Production Using a Gas Chromatography

In order to confirm how much CLA was substantially produced by *Lactobacillus* strains expressing isomerase enzymes, the quantity of generated CLA was determined using a gas chromatography.

### <Materials and Method>

*Lactobacillus* candidates were inoculated into a MRS liquid medium containing LA, prior to culturing the mixture at 37 °C for 24-48 hours. The cultured medium was extracted with heptadecanoic acid and a mixture of chloroform:methanol. The extract was treated with sodium sulfate to remove moisture, and then evaporated. 1N Sodium hydroxide(in methanol) was added to the prepared sample, prior to saponifying the sample at 100 °C for 15 minutes. Thereto 4% HCl(in methanol) was added to be methylated. Hexane:water(1:1, v/v) were added to the methylated sample, and then mixed and centrifuged. An organic solvent fraction was taken to remove organic solvent using nitrogen gas, followed by dissolving the sample in hexane 1mL.

According to the present invention, CLA content within each sample before and after the removal of oxides was measured by gas chromatography(Hewlett Packard 5890 Series II GC) with a flame ionization detector(FID). The used capillary column(DB FFAP capillary column) has a length of 30m, an inner diameter of 0.25µm, and a film thickness of 0.25µm. After setting the column into a GC, a GC was used under the following conditions: oven temperature(210°C); detector temperature(270°C); injector temperature(250°C); carrier gas(Helium(1mL/min)); split ratio(50:1); and sample injection(2µℓ). Each peak area was calculated using an integrator(3395, Hewlett Packard) linked with the GC. CLA was identified, as compared with the retention time of a standard material. Heptadecanoic acid was used as an internal standard material in order to measure CLA contents(Lin, T.Y. 2000. Conjugated linoleic acid concentration as affected by lactic cultures and additives, Food Chemistry 69. 27-31).

### <Results and Discussion>

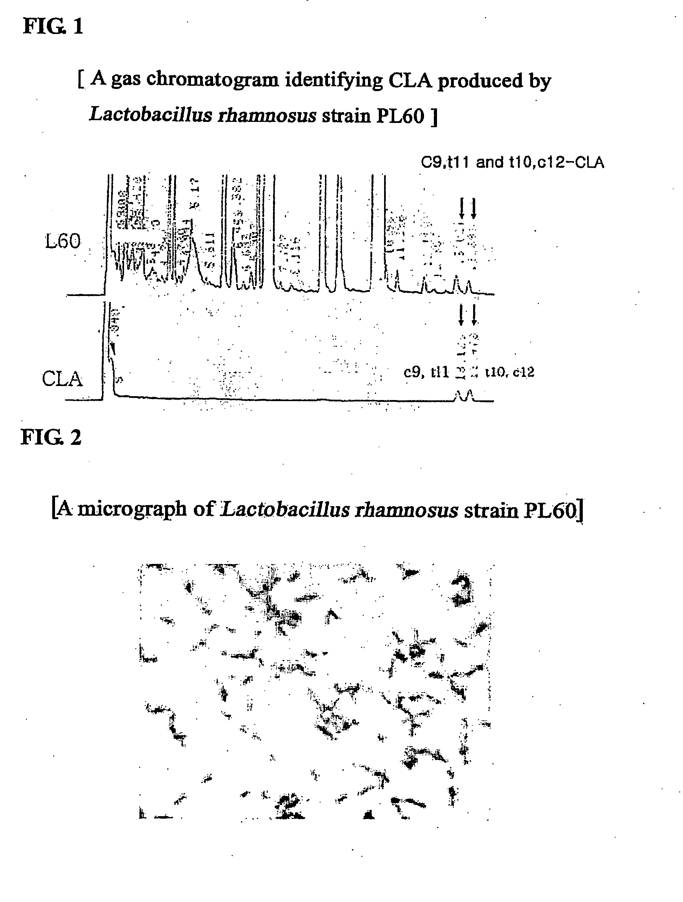

As indicated in the gas chromatogram of Fig. 1, the isolated *Lactobacillus* strain produced both c9t11 and t10c12 isomers of CLA. If yield of t10c12 CLA with a body-fat reducing effect was indicated in terms of ppm, *Lactobacillus rhamnosus* Strain PL60 produced t10c12 CLA in an amount of 43.25ppm and had more excellent productivity in comparison with the reported *Lactobacillus reuteri* and *Lactobacillus fermentum* that produced the CLA in amounts of 30ppm and 28ppm respectively.

### Experimental Example 2: Identification of Lactobacillus strain: Gram's Staining, Identification Using API Kit, 16S rRNA Sequence Analysis. Multiplex PCR Assays

In order to identify CLA-producing *Lactobacillus* strains, it was confirmed whether they showed gram positive on a gram's staining and catalase negative or not. Various biochemical and physiological tests were carried out using an API kit, and 16S rRNA sequence was analyzed and identified. In addition, in order to classify closely related species, strains were identified by multiplex PCR assays using a group-specific primer.

### 1. Gram's Staining

Straining was smeared on a slide and heat-fixed, prior to adding a crystal violet solution thereon to be reacted for about 1 minute. The resulting slide was treated with an iodine solution to wash an excess of dyes, followed by adding iodine thereon to be treated for 1 minute. The resulting slide was decolorized with 95% ethanol for 30 seconds, and then washed with water for 2-3 seconds to remove water with a sucker. The resulting slide was treated with a safranin 0 solution for 10-30 seconds for a counter stain. The resulting slide was carefully rinsed with water until dyes didn't come out any more, followed by drying the resulting slide with a sucker and letting a drop of immersion oils fall to be observed through a microscope.

### <Results and Discussion>

As shown in Fig. 2, CLA-producing *Lactobacillus* stains exhibited gram positive.

### 2. Biochemical and Physiological Tests Using an API Kit

After confirming whether strains were purely isolated or not, the strains were cultured in a MRS medium at 30°C or 37°C for 24 hours. They were more than twice subcultured in a MRS broth, prior to isolating a colony from a MRS medium. A suspension medium ample was opened to prepare a heavy suspension with very high turbidity using a cotton ball. The prepared strain solution was dropped into the suspension medium 5mL drop by drop till turbidity reached McFarland 2. The API 50 CHL medium containing the strains was divided into tubes of a strip and cultured under the aerobic condition at 30° C or 37°C for 48 hours. If acid is generated, an API kit makes a medium yellowish by a bromocresol purple indicator within the medium. If color changes from purple to black in an Esculin test(Tube No. 25), it means a positive reaction.

### <Results and Discussion>

As indicated Table 1, experimental results using an API 50CH kit showed that the *Lactobacillus* strain of the invention had a similarity to *Lactobacillus rhamnosus* and *Lactobacillus para paracasei,* but it wasn't evaluated in terms of %.

**[Table 1]**

| Results on identification of *Lactobacillus* strain using API CH50 kit | | | | |
|---|---|---|---|---|
| Strip No.1 | Strip No.1 | Strip No.1 | Strip No.1 | Strip No.1 |
| Tube/substrate | Tube/substrate | Tube/substrate | Tube/substrate | Tube/substrate |
| -Control | +Galactose | -D-Mannoside | -Melibiose | -D-Turanose |
| -Glycerol | +D-Glucose | -D-Glucoside | -Saccharose | -D-Lyxose |
| -Erythritol | +D-Fructose | +Glucosamine | +Trehalose | +D-Tagatose |
| +D-Arabinose | +D-Mannose | +Amygdaline | -Inuline | -D-Fucose |
| -L-Arabinose | -L-Sorbose | +Arbutine | +Melizitose | +L-Fucose |
| +Ribose | -Rhamnose | +Esculine | -D-Raffinose | -D-Arabitol |
| -D-Xylose | -Dulcitol | +Salicine | -Amidon | -L-Arabitol |
| -L-Xylose | -Inositol | +Cellobiose | -Glycogene | +Gluconate |
| -Adonitol | +Mannitol | -Maltose | -Xylitol | -2-Gluconate |
| -Xyloside | +Sorbitol | -Lactose | +Gentiobiose | -5-Gluconate |
| *Lacto. para. Paracasei 3* | | | | |
| *Lacto. papa. Paracasei 1* | | | | |

### 3. Identification using 16S rRNA sequence analysis

Genomic DNA was isolated to amplify a 16S ribosomal DNA fragments thereof, prior to confirming the amplified DNA fragments by an electrophoresis. DNA fragments were purified using a Qiagen PCR purification kit(Qiagen, Hilden, Germany) to be mixed with a reactant solution containing d-Rhodamine dye-labeling dd-NTP, prior to performing a direct sequencing to purify the obtained DNA using an ethanol/sodium acetate precipitation. The purified DNA was dissolved in TSR(template suppression reagent) to be analyzed with an ABI prism 310 Genetic analyzer(PE Applied Biosystems, U.S.A). The analyzed sequence was identified using Genebank(http://www.ricbi.nlm.nih.gov/).

### <Results and Discussion>

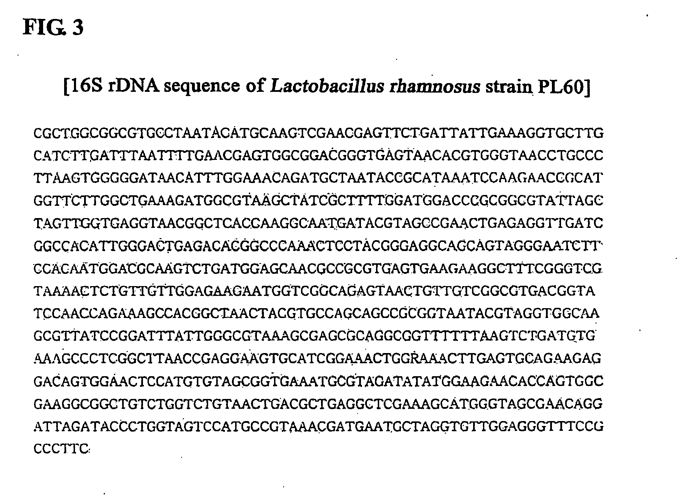

As a result of analyzing the sequence of CLA-producing *Lactobacillus* strain(Fig. 3), it showed a similarity to *Lactobacillus rhamnosus* 842/844(99%) and *Lactobacillus casei(99%).*

### 4. Identification Using Multiplex PCR

In order to confirm whether the PL60 strain was *Lactobacillus rhamnosus* or *Lactobacillus casei*(*Lactobacillus paracasei*)*,* the DNA fragments obtained from the PL60 strain were compared with their DNA fragments after performing multiplex PCR assays(Song, Y., N. Kato, C. Liu, Y. Matsumiya, H. Kato, and K. Watanabe. 2000. Rapid identification of 11 human intestinal Lactobacillus species by multiplex PCR assays using group- and species-specific primers derived from the 16S 23S rRNA intergenic spacer region and its flanking 23S rRNA. FEMS Mictrobiol. Letters, 187:167-173). For this, a PCR reaction was carried out in a final amount of 30µℓ using a mixture containing 1x reaction buffer, dNTPs of 200 µM, Taq polymerase of 0.15 units, primers of 10pmol(LU-5, CTA GCG GGT GCG ACT TTG; Lpar-4, GGC CAG CTA TGT ATT CAC TGA; Rha II , GCG ATG CGA ATT TCT ATT ATT), and DNA of 20ng. The PCR reaction comprised the following steps of: reacting the mixture repeating a cycle consisting of 20 seconds at 95°C, 2 minutes at 62 °C, and 2 minutes at 74°C 35 times; reacting the mixture at 72°C for 10 minutes; and preserving the resultant at 4°C. The PCR resultant was electrophoresed in 1.5% agarose gel for 20 minutes using a 0.5x TBE(0.045M tris-borate, 0.001M EDTA) buffer solution, followed by observing the patterns of the developed DNA fragments.

### <Results and Discussion>

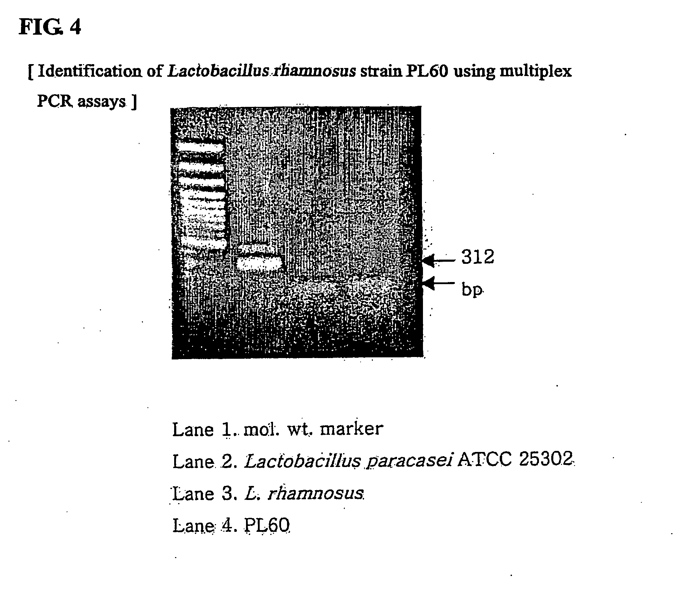

As a result of multiplex PCR assays, *Lactobacillus rhamnosus* strain and the PL60 strain produced DNA fragments of 113bp, whereas *Lactobacillus paracasei* ATCC 25302 produced DNA fragments with a size of 312bp as shown in fig. 4. Consequently, the PL60 strain was identified as *Lactobacillus rhamnosus.* Up to now, CLA-producing *Lactobacillus rhamnosus* strain hasn't been reported yet. The present invention reports CLA-producing *Lactobacillus rhamnosus* strain for the first time.

### Experimental Example 3: Intestinal Adaptation of Lactobacillus rhamnosus

In order to be used as a probiotic, it must have strong tolerance to both acid and bile and good adaptation to intestinal cells. An intestinal adaptation should be confirmed through human experiments.

### 1. Acid Resistance Test

In order to know if pH affected survivability of selected strains, a MRS(DeMan-Rogosa-Sharpe) medium was used after adjusting pH to 7.0, 4.8, and 4.5 using 10N HCl. An activated strain solution(0.D=2.0) was inoculated into a MRS medium in an amount of 2% and cultured at 37°C for 24 hours, prior to measuring an absorbance at 600nm. It was examined if pH affected growth of selected strains using the measured absorbance. The 0.D of pH 7.0 was diluted to 1/10 to measure and record an absorbance(Conway PL, Gorback SL, Goldin BR, 1987. Survival of lactic acid bacteria in the human stomach and adhesion to intestinal cells. J. Dairy Sci. 70:1-12).

### <Results and Discussion>

As a result of an experiment on survivability in the presence of low acid, even if said strains were treated for 24 hours, they survived, hence had a strong resistance to acid as shown in Table 2.

**[Table 2]**

| Experimental results on acid resistance of *Lactobacillus rhamnosus* Strain PL60(0.D. at 600nm) | | | | |
|---|---|---|---|---|
| Time | 0hr | 3hr | 6hr | 24hr |
| PH7.0 | 0.022 | 0.069 | 0.467 | 8.080 |
| PH4.8 | 0.036 | 0.063 | 0.249 | 6.850 |
| PH4.5 | 0.029 | 0.064 | 0.200 | 5.820 |

### 2. Bile Resistance Test

In order to know if bile affected growth of selected strains, ox-gall(OXOID) was added to a MRS(DeMan-Rogosa-Sharpe) medium in amounts of 0.125% and 0.25% to be sterilized. The activated strain solution(0.D=2.0) was inoculated into the sterilized medium in an amount of 2% and cultured at 37°C for 24 hours, followed by measuring an absorbance at 600nm. The 0.D in 0% bile was diluted to 1/10 to measure and record an absorbance(Ibrahim SA, Bezkorovainy A. 1993. Survival of bifidobacteria in the presence of bile salt. J. Sci. Food Agric. 62: 351-354).

### <Results and Discussion>

Healthy people have a bile concentration of 0.06% within the small intestines. The strains survived even in the presence of 0.250% bile, thus had a strong bile resistance.

**[Table 3]**

| Time | 0hr | 3hr | 6hr | 24hr |
|---|---|---|---|---|
| Bile 0.000% | 0.022 | 0.069 | 0.467 | 8.080 |
| Bile 0.250% | 0.007 | 0.038 | 0.335 | 3.570 |

### 3. Intestinal Adhesion Test

In order to know an adhesion to the human intestines, *Lactobacillus rhamnosus* Strain PL60 was adhered to Caco-2 cell lines derived from intestinal epidermal cells. For this, Caco-2 cell lines were cultured in a DMEM medium(pH 7.0) containing sodium bicarbonate 2.7g/L, 20%(v/v) fetal bovine serum(FBS) and antibiotics antimicotics. 3x10⁵ Cells were inoculated into a medium of 2mL in a petri dish of 30mm to be cultured into a single layer. The medium was changed once every two days. The cell single layer was twice rinsed with a phosphate buffered saline(PBS) solution of 2mL, 6 days later. The *Lactobacillus* strain of 1x10⁷ cells was suspended in a medium of 2mL and added to a petri dish, prior to culturing the admixture at 37°C under an 5% CO₂-95% air atmosphere for 60-90 minutes. The cells were twice rinsed with a sterilized PBS and fixed with methanol for 10 minutes. They were observed through an optical microscope after a gram's stain. 20 Fields were inspected under a 100-fold microscope for a quantitative analysis. The number of adhered strains was counted and indicated in terms of the number of adhered strains per 100 Caco-2 cells(Bibiloni R, Perez PF, DeAntoni GL. 1999. Anaerobe 5, 483-485; Edited by R Fuller (1997) Probiotics 2, 10-22).

### <Results and Discussion>

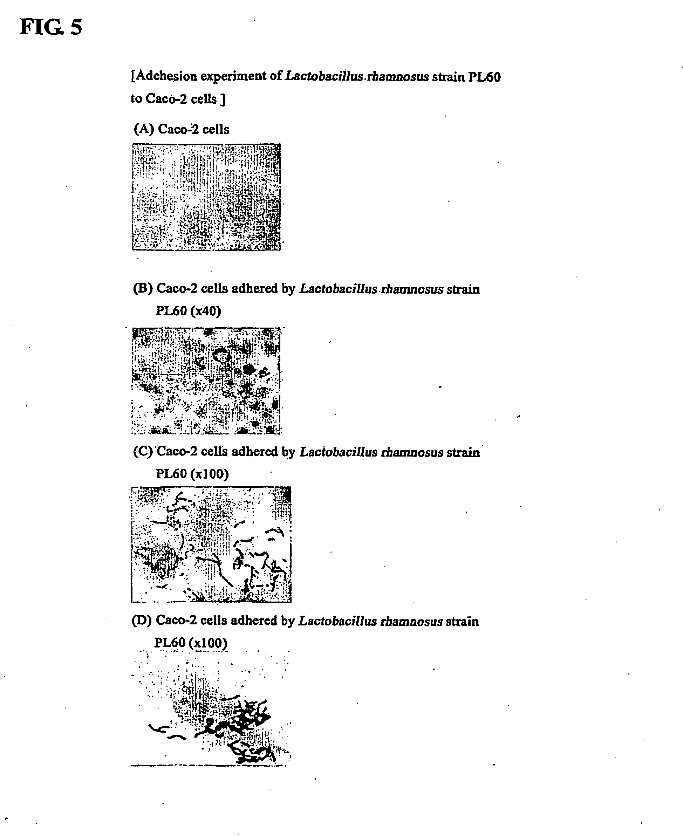

As shown in Fig. 5, *Lactobacillus rhamnosus* Strain PL60 has excellent adhesion to the Caco-2 cells. If the number of adhered strains per a field was counted out of 20 fields to calculate an average number of adhered strains per a field, 59.29±5.33 *Lactobacillus* strains per a field were adhered. This means that more than 4000 of *Lactobacillus* strains per a petri dish were adhered to the cells and had better intestinal adhesion than the conventional *Lactobacillus* strains.

### 4. Adaptation Test to the Human Intestines

In order to confirm whether *Lactobacillus* strains were adapted to the intestines after people substantially took them, *Lactobacillus rhamnosus* Strain PL60 was orally administered in an amount of 10¹⁰ CFU once a day for 8 days. The next day, feces were cultured in a MRS(with 1% bromo phenol blue, 30µg/mL vancomycin) for 48 hours. All the similar colonies were examined by a gram's stain, subcultured, and purely isolated. Species-specific PCR assays were carried out using purely isolated colonies.

### <Results and Discussion>

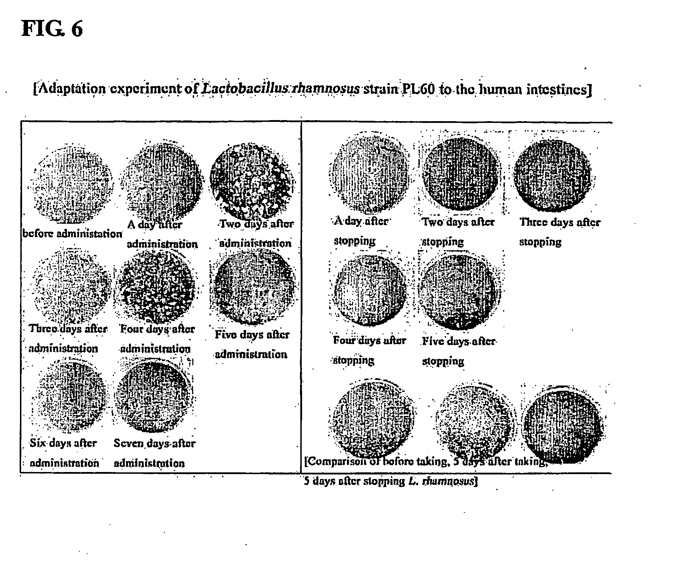

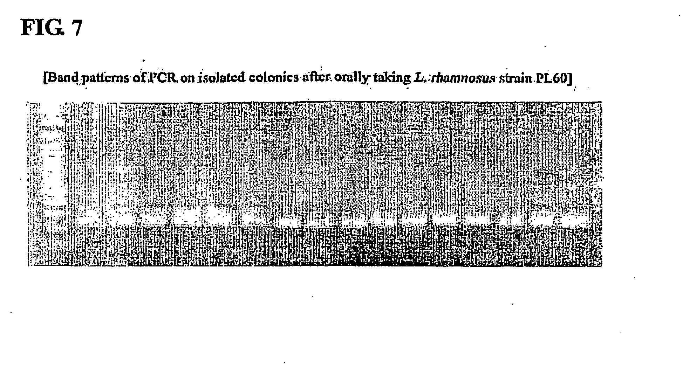

As shown in Fig. 6, *Lactobacillus rhamnosus* Strain PL60 had been detected from one day to the six days after taking it and stopped an administration as soon as it was detected. The detected *Lactobacillus* colony turned out to be *Lactobacillus rhamnosus* by a species-specific PCR assay(Fig. 7). This proves that *Lactobacillus rhamnosus* Strain PL60 was adapted to the intestines. Especially, as shown in Fig. 6, it was thought that judging by the fact that bacterial florae within the intestines got simpler after *Lactobacillus rhamnosus* Strain PL60 was administered, the *Lactobacillus* strain had an intestinal regulation.

### Experimental Example 4: Safety Test of Lactobacillus strains

The safety test of *Lactobacillus* strains should be carried out for human dosage. For this, it was confirmed whether *Lactobacillus* strains produced toxic materials, such as ammonia, indole, hemolysin or like or not, and poisonous enzymes were present or not.

### 1. Hemolysis Test

When *Lactobacillus rhamnosus* Strain PL60 was inoculated into a sheep blood agar and cultured at 37°C for 24 hours, only α-hemolysis was found; not β-hemolysis.

### 2. Gelatin Liquefaction Test

*Lactobacillus rhamnosus* Strain PL60 was inoculated into a slant medium made of a MRS gelatin medium(beef extract of 0.3g, peptone of 0.5g, gelatin of 12g, and MRS broth of 100mL) and cultured at 35 °C for 6 weeks. When it, together with a control, was cooled at 4°C for 4 hours or so to examine gelatin liquefaction, it was thought that gelatinases were not present because a gelatin liquefaction wasn't observed.

### 3. Ammonia Formation Test

A urea agar medium(urea of 20g, NaCl of 5g, KH₂PO₄ of 2g, peptone of 1g, glucose of 1g, phenol of 12mg, and distilled water of 100mL) was filtered and sterilized, followed by dissolving agar of 15g in distilled water of 900mL to be wet-sterilized and mixed with the prepared urea agar medium to adjust a total volume to 1L(pH 6.9). Thereto *Lactobacillus rhamnosus* Strain PL60 was inoculated and cultured at 37°C for 12 hours or so, prior to observing color change of the medium. Because a yellow medium means negative, it was proved that *Lactobacillus rhamnosus* Strain PL60 didn't generate ammonia.

### 4. Indole Formation Test

*Lactobacillus rhamnosus* Strain PL60 was inoculated into a MRS agar containing 0.1% tryptone and cultured for 18 hours or so. When thereto 5 drops of a Kovac's reagent(p-dimethylaminobenzaldehyde of 10g, buthanol of 150mL, and hydrocholic acid of 50mL) were added, there was no color change. This means that indole wasn't produced.

### 5. Phenylalanine Deamination Test

*Lactobacillus rhamnosus* Strain PL60 was inoculated into a MRS medium containing 0.2% D,L-phenylalanine and cultured for 24 hours or like. After thereto letting 5-10 drops of 10% ferric chloride fall to flow down on a slant medium, a color change was observed within 1-5 minutes. In case of a positive reaction, the generated phenylpyruvic acid was reacted with ferric chloride to make a medium green. *Lactobacillus rhamnosus* Strain PL60 showed a negative reaction.

### 6. β-Glucuronidase Test

p-Nitrophenyl- β-D-glucuronide was dissolved in 0.1M sodium phosphate buffer(pH 6.0) for a 0.2% concentration. *Lactobacillus rhamnosus* Strain PL60 was suspended in a phosphate buffer to Ab₆₀₀=4 to form a suspension. A buffer solution of 200µℓ with a substrate was added to the suspension of 200µℓ and treated at 37°C for 16 hours. If a culture solution gets yellow, it is positive. However, this test showed a negative reaction. The culture solution was centrifuged to take a supernatant. When an absorbance of the supernatant was determined at 405nm, it was 0.078.

### 7. Nitroreductase Activity Test

*Lactobacillus rhamnosus* Strain PL60 cultured in a MRS liquid medium overnight was centrifuged at 3000xg for 10 minutes to collect biomass, prior to sonicating the biomass for 5 minutes. 4-Nitrobenzoic acid(final conc. 30µg/mL) and trichloroacetic acid(final conc. 0.21%) were added to the supernatant and treated at 37°C for 1 hour, followed by adding sodium nitrite(final conc. 0.007%) to be treated at room temperature for 20 minutes. Thereto ammonium sulfamate(final conc. 0.04%) was added and treated at room temperature for 3 minutes. Thereto NEDD(N-(1-naphtyl)ethylenediamine dihydrochloride)(final conc. 0.35%) was added and developed at 4°C. When the developed supernatant was observed under a 540nm spectrophotometer, it showed a negative reaction. It was compared with a positive reaction obtained from adding 4-aminobenzoic acid of 1µg/mL.

### 8. Antibiotic Resistance

The stronger antibiotic resistance a probiotic has, the higher survivability within the intestines is. Thus, the stronger an antibiotic resistance is, the better it is. However, if an antibiotic resistance is transferred, resistance problems may be brought about. It was confirmed whether an antibiotic resistance was transferred to other bacteria or not.

**[Table 4]**

| Antibiotic resistance of *Lactobacillus rhamnosus* Strain PL60 | |
|---|---|
| Antibiotic | Diameter(mm) of growth inhibition |
| Ampicillin | 21 |
| Carbenicillin | 25 |
| Cefoperazone | 22 |
| Cephalothin | 20 |
| Chloramphenicol | 24 |
| Clindamycin | 26 |
| Erythromycin | 28 |
| Gentamicin | 10 |
| Oxacillin | 8 |
| Penicillin | 25 |
| Piperacilin | 30 |
| Rifampin | 24 |
| Streptomycin | 11 |
| Tetracycline | 29 |
| Trimethpprime/sulfamethoxasole | 6 |
| Vancomycin | 6 |

### 9. Transfer Test of Antibiotic Resistance

In order to examine the transfer of an antibiotic resistance, a filter binding assay was carried out(Givers, D., G. Huys, and J, Swings. 2003. In vitro conjugal transfer of tetracycline resistance from Lactobacillus isolates to other Grain-positive bacteria. FEMS Microb. Letters 225:125-130). *Lactobacillus rhamnosus* Strain PL60 was cultured to a mid-exponential phase(approximately 4-5 hours). The cultured strain of 1mL was mixed with *Enterococcus faecalis* CCARM 5510 of 1mL, followed by filtering the mixture through a sterilized cellulose acetate filter to be washed with PPS(peptone physiological saline solution). The filter paper was put on a non-selective agar medium and cultured at 37°C for 16 hours. Biomass grown on the filter paper was washed with PPS of 2mL and detached from the paper, prior to diluting the biomass to be inoculated into an Enterococcosal selective medium containing various antibiotics and cultured at 37°C for 24-48 hours. It was examined whether *E. faecalis* with an antibiotic resistance was present or not, but there was no *E. faecalis* with an antibiotic resistance in the culture. This means that the antibiotic resistance was not transferred.

### [Mode for Invention]

### Example 2: Optimum Conditions for Producing CLA

We found the concentration of LA and the kind of substrates that can maximally produce CLA.

### 1. LA Concentration Capable of Producing Maximum CLA

As LA of high concentration inhibits the growth of bacteria themselves, LA can't be added to a medium in high concentration. In addition, in order to save LA spent on a medium LA concentration that could produce maximum CLA was found out.

### <Materials and Method>

Water-soluble LA ester was added to a skim milk medium and MRS medium for various concentrations and cultured overnight, followed by measuring the quantity of CLA generated within the media. For this, lipid within a medium was extracted and methylated, prior to measuring the quantity of generated CLA using a GC. To do this, heptadecanoic acid of 1000 ppm and chloroform:methanol(2:1) of 200mL were added to a culture solution of 20mL, followed by thereto adding glass beads to be strongly shaken for 5 minutes and homogenized for 5 minutes.

The admixture was centrifuged at 6000rpm for 15 minutes(4°C) and separated into two fractions. An organic solvent fraction was treated with sodium sulfate to remove residual moisture, prior to evaporating organic solvent to be dried with nitrogen gas. 1N Sodium hydroxide(methanol) of 3mL was added to the dried sample and saponified at 100 °C for 15 minutes. At this time, a screw-capped tube treated with a Teflon tape was used and the cap was wrapped with a parafilm. Thereto 4% HCl(methanol) of 6mL was added to be methylated for 20 minutes. The methylated sample was mixed with hexane:water(1:1, v/v) of 2mL and strongly shaken for 10 minutes, followed by centrifuging the mixture at 8000rpm and 4°C for 15 minutes. An organic solvent fraction was taken and dried using nitrogen gas, prior to dissolving the dried matter in hexane 1mL.

### <Results and Discussion>

Supposing that the peak area of heptodecanoic acid, a standard reference material, is 100, when LA was in an amount of more than 100ppm added to a medium CLA was produced in a sufficient amount(Table 5). In addition, if LA was in amounts of 1000ppm and 500ppm each added there was no striking difference between them in producing CLA. Preferably, LA is in an amount of 100-1000ppm added in order to produce CLA. In the view of cost and efficiency, 500ppm is most preferable.

**[Table 5]**

| CLA production according to LA concentration added to a medium | | | | | | |
|---|---|---|---|---|---|---|
| Retention time(min.) | | LA concentration added to medium(ppm) | | | | |
| | | 0 ppm | 10 ppm | 100 ppm | 500 ppm | 1000 ppm |
| 6.867 | Heptadecanoic acid | 100 | 100 | 100 | 100 | 100 |
| 12.002 | CLA(c9, t11) | 12690 | 12730 | 14850 | 15250 | 15779 |
| 12.332 | CLA(t10, c12) | **3984** | **3990** | **5435** | **8854** | **9727** |
| 13.000 | | 8211 | 8158 | 8148 | 8639 | 8573 |

### 2. Emulsifier Addition Conditions for Producing Maximum CLA

It was examined if when an emulsifier was added in order to increase the solubility of LA in a culture solution, the production of CLA increased or not. For this, LA was added to a skim milk medium and MRS medium for a 0.1 % concentration. At this time, LA was added in three form of LA, LA salt, and LA and Tween-800(0.2%) and cultured overnight, followed by confirming the CLA productivity of *Lactobacillus rhamnosus* Strain PL60. Using the above-mentioned method, lipid within a culture solution was extracted to be methylated, prior to determining the quantity of CLA by GC.

### <Results and Discussion>

A Tween-80 that was used in order to enhance a solubility of LA in a culture solution tripled the production of t10c12 CLA, as compared with a LA salt. It is very important that an emulsifier was added to enhance solubility of LA upon adding LA to a medium.

**[Table 6]**

| Influence of Tween-80 addition on CLA production | | | | |
|---|---|---|---|---|
| | | *Lactobacillus rhamnosus* Strain PL60 | | |
| RT(min) | Skim milk(blank) | Skim milk | Skim milk+LA salt | Skim milk+LA+Tween 80 |
| 6.859 | 414941 | 406823 | 455181 | 448288 |
| 12.010 | 8414 | 13296 | 19408 | 25817 |
| 12.328 | 2323 | 3797 | 4396 | 12559 |
| 13.000 | 4505 | 7463 | 11330 | 13728 |

### 3. Emulsifier Addition Conditions upon Primary culture for Inducing CLA production

In order to produce maximum CLA immediately after taking *Lactobacillus rhamnosus* Strain PL60 itself, or a starter strain or additive thereof, it was examined whether in case *Lactobacillus rhamnosus* Strain PL60 was cultured to produce products like lyophilized-dry powders, adding Tween-80 to increase solubility of LA was an efficient condition or not. For this, LA salt, LA and Tween-80 of 0.1%, LA and Tween-80 of 0.2%, and LA and Tween-80 of 0.5% were added to a medium on primary-culturing starter strains. The primary-cultured *Lactobacillus rhamnosus* Strain PL60 was cultured in a CLA-producing medium(skim milk containing LA of 0.1%) to measure the quantity of the generated CLA.

### <Results and Discussion>

In order to produce maximum CLA in a skim milk medium(whey medium) used in a commercial production, in case *Lactobacillus rhamnosus* Strain PL60 was cultured in a skim milk medium containing LA of 0.1% and Tween-80 of 0.1-0.5% to induce productivity of CLA, CLA productivity was best(Table 7). It was thought that the reason why 0.2% Tween-80 has higher CLA productivity than 0.5% Tween-80 was the growth inhibition of *Lactobacillus* strains by 0.5% Tween-80.

**[Table 7]**

| CLA productivity of *Lactobacillus rhamnosus* Strain PL60 depending on concentrations of Tween-80 for dissolving LA in a medium | | | | | | |
|---|---|---|---|---|---|---|
| | Control (without LA) | Skim milk containing 0.1 % LA | | | | |
| | | LA salt | LA+Tween8 0(0.01%) | LA+Tween8 0(0.1%) | LA+Twee n80(0.2%) | LA+Twee n80(0.5%) |
| 6.859 | 100 | 100 | 100 | 100 | 100 | 100 |
| 12.010 | 5980 | 6901 | 12846 | 15972 | 18610 | 14886 |
| c9t11 | | | | | | |
| 12.320 | 2398 | 3229 | 4110 | 4184 | 6562 | 4006 |
| t10c12 | | | | | | |
| 13.000 | 4450 | 5161 | 10313 | 8912 | 10183 | 8786 |

### 4. Saccharide-Addition Conditions for Producing Maximum CLA

We found out the kind of saccharides capable of producing maximum CLA. To do this, fructose, sucrose, and lactose each was added to a skim milk containing 0.1 % LA medium for a 6% concentration to measure a production of CLA.

### <Results and Discussion>

CLA was produced most on adding fructose, followed by sucrose and lactose. When glucose was added, an effective CLA production was not observed.

**[Table 8]**

| Change of CLA production depending on various saccharides | | | | | | |
|---|---|---|---|---|---|---|
| | Skim milk | | | | | |
| RT | without PL60 | Control | lactose | fructose | glucose | sucrose |
| 6.895 | 30968 | 459810 | 496664 | 447001 | 381032 | 468243 |
| 12.091 | 28760 | 40559 | 44592 | 46703 | 33612 | 43901 |
| 12.419 | 17850 | 24097 | 24201 | 29864 | 14860 | 23519 |

### Example 3: Change of the Body Weight of Rats Administered with CLA-Producing Lactobacillus rhamnosus Strain PL60

A lyophilized *Lactobacillus rhamnosus* Strain PL60 that was cultured in a medium containing 0.1 % LA and 0.2% Tween-80 using skim milk as an excipient was administered into a rat in a dose of 10⁹ CFU/day and 10⁷ CFU/day with giving a high-fat diet, followed by observing the change of body weight of a rat.

### <Materials and Method>

Four C57BL/6N rats(Charles river laboratory animal facility, USA) were assigned to five groups. The first group was a group administered with a normal diet(Purina rodent chow #5057(3.28cal/g), the second group was a group administered with a high-fat diet(Research diet 45% high fat diet D12451(5.252cal/g), the third group was a control group administered with a high-fat diet and skim milk of an excipient, the fourth group was a group administered with a high-fat diet and *Lactobacillus rhamnosus* Strain PL60 in high concentration(10⁹ CFU/day), and the fifth group was a group administered with a high-fat diet and *Lactobacillus rhamnosus* Strain PL60 in low concentration(10⁷ CFU/day). While 3 week-old rats ate a high-fat diet and water to the full, the change of their body weight and the quantity of a fed diet were observed. The rats were anatomized on the 8th week to observe weight of intestinal fat, the size and number of adipose cells in all organs using a microscope after a stain.

### <Results and Discussion>

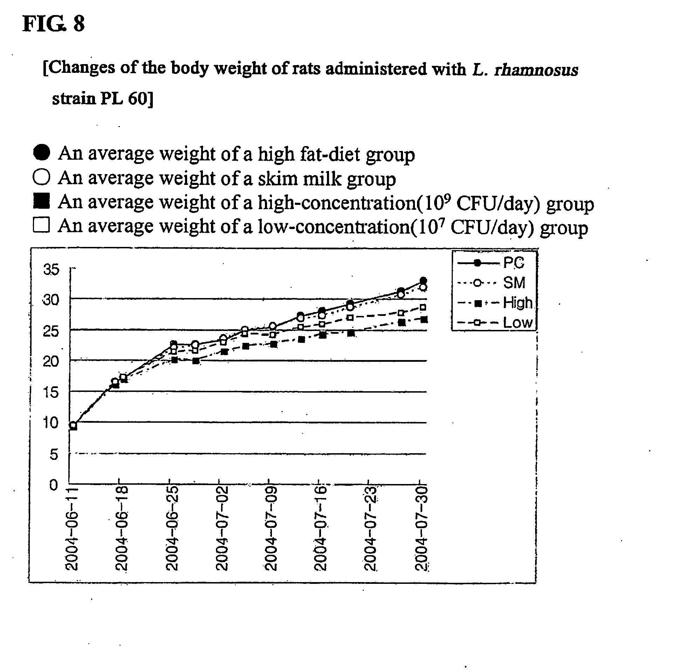

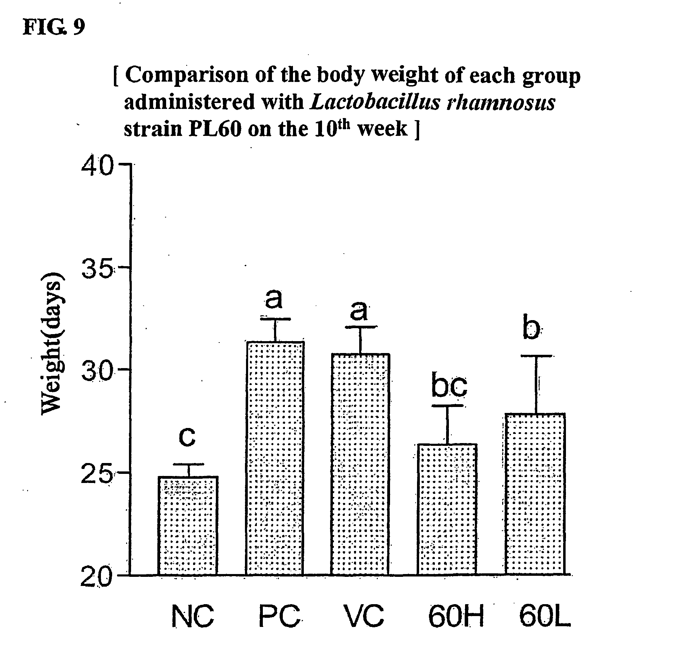

Table 9 represents the change of body weight of rats administered with *Lactobacillus rhamnosus.* Strain PL60. According to Table 9, while a group administered with *Lactobacillus rhamnosus* Strain PL60 in high concentration, hardly showed a significant statistic on the 4th week, it had lower weight gain by more than 2g on the 8th week, as compared with a control group(Fig. 8 and Fig. 9). That is to say, a normal-diet group had an average weight of 24.7g, a high fat-diet group had an average weight of 33.4g, a skim-milk group had an average weight of 31.9g, a group administered with *Lactobacillus rhamnosus* Strain PL60 in high concentration had an average weight of 26.9g, and a group administered with *Lactobacillus rhamnosus* Strain PL60 in low concentration had an average weight of 28.7g. The weight gain of the high-concentration group was lower than that of the high fat-diet group by 6.5g, which was 19.5%. The low-concentration group had a lower weight gain than the high fat-diet group by 4.7g, which was 14%. The high-concentration group and low-concentration group respectively showed lower weight gain by 5g(15.7%) and 3.2g(10%), as compared with a skim milk group. It was thought that the weight difference between a high fat diet group and a skim milk group was 1.5g(4.5%), which was in the tolerance error range and was not a weight loss resulting from skim milk.

**[Table 9]**

| | No. | 6/11 | 6/17 | 6/18 | 6/25 | 6/28 | 7/2 | 7/5 | 7/9 | 7/13 | 7/16 | 7/20 | 7/27 | 7/30 |
|---|---|---|---|---|---|---|---|---|---|---|---|---|---|---|
| | | 3weeks | | | | | | | | | | | | |
| NC | 1 | 9.8 | 14.8 | 16 | 19.9 | 20 | 22 | 22 | 23 | 22.3 | 23 | 22.1 | 24.2 | 23.5 |
| | 2 | 9 | 123. | 15 | 20.8 | 21.8 | 22 | 22 | 23 | 23.2 | 23.5 | 24.5 | 25.5 | 25.5 |
| | 3 | 9 | 14.2 | 13 | 19.1 | 19.9 | 21 | 22 | 23 | 23.1 | 23.6 | 24.3 | 25.1 | 25.3 |
| | 4 | 9.2 | 14.6 | 14 | 19.2 | 19.8 | 21 | 21 | 21 | 21.5 | 21.9 | 23 | 24.3 | 24.5 |
| | Average | 9.3 | 14 | 14 | 19.8 | 20.4 | 22 | 22 | 22 | 22.5 | 23 | 23.5 | 24.8 | 24.7 |
| PC | 1 | 8.9 | 16 | 16 | 20.9 | 21.7 | 23 | 24 | 24 | 25.5 | 26.5 | 27.5 | 29.7 | 31.6 |
| | 2 | 10.1 | 16.8 | 17 | 23.5 | 22.9 | 23 | 25 | 26 | 27.3 | 27.8 | 29.3 | 31.6 | 33.5 |
| | 3 | 9.8 | 17.2 | 18 | 21.9 | 22.6 | 23 | 25 | 25 | 27.3 | 28.2 | 29.4 | 31.8 | 33.6 |
| | 4 | 9 | 15.7 | 17 | 24.1 | 23.7 | 25 | 26 | 27 | 29.3 | 29.5 | 31.1 | 32.2 | 33.4 |
| | Average | 9.5 | 16.4 | 17 | 22.6 | 22.7 | 24 | 25 | 26 | 27.4 | 28 | 29.3 | 31.3 | 33 |
| SM | 1 | 8.2 | 14.6 | 16 | 21.4 | 22.1 | 23 | 24 | 25 | 26.3 | 27.1 | 27.8 | 29.5 | 30.4 |
| | 2 | 10 | 17.1 | 18 | 21.2 | 21.4 | 23 | 24 | 24 | 25.3 | 25.6 | 27.2 | 29.7 | 31.1 |
| | 3 | 9.3 | 16.8 | 18 | 23.3 | 23.4 | 25 | 26 | 27 | 28 | 28.6 | 29.5 | 31.6 | 32.8 |
| | 4 | 10.3 | 17.3 | 18 | 22.5 | 23.3 | 24 | 26 | 27 | 27.7 | 28 | 30.1 | 32.1 | 33.3 |
| | Average | 9.5 | 16.5 | 17 | 22.1 | 22.6 | 24 | 25 | 26 | 26.8 | 27.3 | 28.7 | 30.7 | 31.9 |
| high | 1 | 9 | 15.3 | 16 | 19 | 19.6 | 21 | 22 | 22 | 22.8 | 23.8 | 23.8 | 24.6 | 25.1 |
| | 2 | 9.2 | 16.1 | 17 | 21.8 | 21.2 | 23 | 25 | 25 | 24.2 | 25 | 26.5 | 28.6 | 29.5 |
| | 3 | 9.4 | 16.4 | 17 | 20.3 | 19.6 | 21 | 22 | 22 | 24.1 | 24.7 | 25 | 27.2 | 27.7 |
| | 4 | 9.1 | 16.2 | 18 | 20 | 19.5 | 21 | 22 | 23 | 23.2 | 23.7 | 23.1 | 25.1 | 25.3 |
| | Average | 9.2 | 16 | 17 | 20.3 | 20 | 22 | 23 | 23 | 23.6 | 24.3 | 24.6 | 26.4 | 26.9 |
| low | 1 | 8.8 | 15.6 | 17 | 23.5 | 22 | 25 | 26 | 26 | 27.1 | 28.1 | 28.5 | 27 | 29.2 |
| | 2 | 9.9 | 16.7 | 18 | 21.1 | 22.2 | 23 | 26 | 26 | 26.5 | 26.5 | 28.1 | 31.2 | 32.1 |
| | 3 | 9.5 | 17.4 | 18 | 21.3 | 22 | 23 | 24 | 24 | 25.6 | 26.5 | 27.3 | 28.6 | 28.6 |
| | 4 | 9.4 | 16.3 | 17 | 20 | 20.2 | 22 | 22 | 22 | 22.5 | 22.7 | 23.9 | 24.5 | 24.9 |
| | average | 9.4 | 16.5 | 17 | 21.5 | 21.6 | 23 | 24 | 24 | 25.4 | 26 | 27 | 27.8 | 28.7 |

### [Industrial Applicability]

*Lactobacillus rhamnosus* Strain PL60 of the present invention has a body-fat reducing effect. Said *Lactobacillus* strain can be directly used as body-fat reducing functional foods for preventing or treating all diseases resulting from obesity, or can be used as additives of body-fat reducing functional foods.

## Claims

1. A *Lactobacillus rhamnosus* strain PL60 KCCM-10654P converting linoleic acid (LA) into conjugated linoleic acid (CLA).

2. The *Lactobacillus rhamnosus* strain PL60 KCCM-10654P of claim 1, wherein said strain is a live strain or dried strain.

3. A composition for producing CLA comprising the strain *Lactobacillus rhamnosus* strain PL60 KCCM-10654P according to claim 1 or 2.

4. A mass-producing process of CLA using *Lactobacillus rhamnosus* strain PL60 KCCM-10654P primary-culturing the strain according to one of claims 1 to 3.

5. The mass-producing process of CLA using *Lactobacillus rhamnosus* strain PL60 KCCM-10654P as set forth in claim 4, wherein 0.01-1.0% LA is added to a primary-culture medium of strain.

6. The mass-producing process of CLA using *Lactobacillus rhamnosus* strain PL60 KCCM-10654P as set forth in claim 5, wherein 0.01-1.0% Tween-80 is added to a primary-culture medium of strain.

7. The mass-producing process of CLA using *Lactobacillus rhamnosus* strain PL60 KCCM-10654P as set forth in claim 6, wherein fructose, sucrose, or lactose as a carbohydrate substrate is added to a primary-culture medium of strain.

8. Body-fat reducing functional foods comprising as an additive a *Lactobacillus rhamnosus* strain PL60 KCCM-10654P of claim 1 or 2, converting linoleic acid (LA) into conjugated linoleic acid (CLA).

9. The body-fat reducing functional foods as set forth in claim 8, wherein foods are health care foods or fermented foods containing yogurt, cheese, kimchi, kochujang (Korean thick soy paste mixed with red pepper), and doenjang (Koean fermented soy paste).

10. Dairy products comprising *Lactobacillus rhamnosus* Strain PL60 KCCM-10654P.

11. Fermented foods from cereals comprising *Lactobacillus rhamnosus* Strain PL60 KCCM-10654P.

12. A medicament comprising live strains, dried strains, or cultural filtrates of *Lactobacillus rhamnosus* Strain PL60 KCCM-10654P.

13. A medicament for use in preventing and treating obesity-related diseases comprising live strains, dried strains, or cultural filtrates of *Lactobacillus rhamnosus* Strain PL60 KCCM-10654P.

14. The medicament for use in preventing and treating obesity-related diseases as set forth in claim 13, wherein healthy people with an average weight of 60kg take *Lactobacillus rhamnosus* Strain PL60 KCCM-10654P in an amount of 1x10⁷-1x10¹¹ CFU per a dose 1-2 times a day.

## Patentansprüche

1. *Lactobacillus* rhamnosus-Stamm PL60 KCCM-1 0654P, welcher Linolsäure (LA) in konjugierte Linolsäure (CLA) umwandelt.

2. *Lactobacillus rhamnosus-*Stamm PL60 KCCM-10654P nach Anspruch 1, wobei der Stamm ein lebender Stamm oder ein getrockneter Stamm ist.

3. Zusammensetzung zur Herstellung von CLA, umfassend den Stamm *Lactobacillus rhamnosus* PL60 KCCM-10654P nach Anspruch 1 oder 3.

4. Massenherstellungsverfahren von CLA unter Verwendung von Primärkultivierung des *Lactobacillus rhamnosus-Stamms* PL60 KCCM-10654P nach einem der Ansprüche 1 bis 3.

5. Massenherstellungsverfahren von CLA unter Verwendung des in Anspruch 4 dargelegten *Lactobacillus rhamnosus*-Stamms PL60 KCCM-10654P, wobei 0,01 -1,0% LA zu einem Primärkulturmedium des Stammes zugegeben wird.

6. Massenherstellungsverfahren von CLA unter Verwendung des in Anspruch 5 dargelegten *Lactobacillus rhamnosus-Stamms* PL60 KCCM-10654P, wobei 0,01-1,0% Tween-80 zu einem Primärkulturmedium des Stammes zugegeben wird.

7. Massenherstellungsverfahren von CLA unter Verwendung des in Anspruch 6 dargelegten *Lactobacillus rhamnosus*-Stamms PL60 KCCM-10654P, wobei Fructose, Sucrose oder Laktose als Kohlenhydratsubstrat zu einem Primärkulturmedium des Stammes zugegeben wird.

8. Körperfettreduzierende funktionelle Nahrungsmittel, umfassend einen Zusatz eines *Lactobacillus rhamnosus*-Stamms PL60 KCCM-10654P nach Anspruch 1 oder 2, welcher Linolsäure (LA) in konjugierte Linolsäure (CLA) umwandelt.

9. Körperfettreduzierende funktionelle Nahrungsmittel nach Anspruch 8, wobei die Nahrungsmittel Gesundheitsnahrungsmittel sind oder fermentierte Nahrungsmittel sind, die Joghurt, Käse, Kimchi, Kochujang (koreanische mit Chilipulver vermischte dicke Sojapaste) und Doenjang (koreanische fermentierte Sojapaste) enthalten.

10. Diätprodukte, umfassend den *Lactobacillus rhamnosus-Stamm* PL60 KCCM-10654P.

11. Fermentierte Nahrungsmittel aus Getreide, umfassend den *Lactobacillus rhamnosus-*Stamm PL60 KCCM-10654P.

12. Medikament, umfassend lebende Stämme, getrocknete Stämme oder Kulturfiltrate vom *Lactobacillus rhamnosus-Stamm* PL60 KCCM-10654P.

13. Medikament zur Verwendung bei der Vorbeugung oder Behandlung von mit Fettleibigkeit verbundenen Krankheiten, umfassend lebende Stämme, getrocknete Stämme oder Kulturfiltrate vom *Lactobacillus rhamnosus-Stamm* PL60 KCCM-10654P.

14. Medikament zur Verwendung bei der Vorbeugung oder Behandlung von mit Fettleibigkeit verbundenen Krankheiten nach Anspruch 13, wobei gesunde Leute mit einem Durchschnittsgewicht von 60kg *Lactobacillus rhamnosus*-Stamm PL60 KCCM-10654P in einer Menge von 1x10⁷ bis 1x10¹¹ CFU für eine Dosis von 1-2 mal am Tag einnehmen.

## Revendications

1. Souche de *Lactobacillus rhamnosus* PL60 KCCM-10654P convertissant l'acide linoléique (LA) en acide linoléique conjugué (CLA).

2. Souche de *Lactobacillus rhamnosus* PL60 KCCM-10654P selon la revendication 1, où ladite souche est une souche vivante ou une souche séchée.

3. Composition destinée à la production de CLA comprenant la souche de *Lactobacillus rhamnosus* PL60 KCCM-10654P selon la revendication 1 ou 2.

4. Procédé de production industrielle de CLA utilisant la souche de *Lactobacillus rhamnosus* PL60 KCCM-10654P par culture primaire de la souche selon l'une quelconque des revendications 1 à 3.

5. Procédé de production industrielle de CLA utilisant la souche de *Lactobacillus rhamnosus* PL60 KCCM-10654P selon la revendication 4, où 0,01 à 1,0% de LA est ajouté à un milieu de culture primaire de la souche.

6. Procédé de production industrielle de CLA utilisant la souche de *Lactobacillus rhamnosus* PL60 KCCM-10654P selon la revendication 5, où 0,01 à 1,0% de Tween-80 est ajouté au milieu de culture primaire de la souche.

7. Procédé de production industrielle de CLA utilisant la souche de *Lactobacillus rhamnosus* PL60 KCCM-10654P selon la revendication 6, où du fructose, du saccharose ou du lactose en tant que substrat d'hydrate de carbone est ajouté à un milieu de culture primaire de la souche.

8. Aliments fonctionnels réduisant les lipides corporels comprenant comme additif une souche de *Lactobacillus rhamnosus* PL60 KCCM-10654P selon la revendication 1 ou 2, convertissant l'acide linoléique (LA) en acide linoléique conjugué (CLA).

9. Aliments fonctionnels réduisant les lipides corporels selon la revendication 8, où les aliments sont des aliments de soins de santé ou des aliments fermentés contenant du yaourt, du fromage, du kimchi, du gochujang (pâte de soja épaisse coréenne mélangée avec des piments rouges) et du doejang (pâte de soja fermentée coréenne).

10. Produits laitiers comprenant la souche de *Lactobacillus rhamnosus* PL60 KCCM-10654P.

11. Aliments fermentés provenant de céréales comprenant la souche de *Lactobacillus rhamnosus* PL60 KCCM-10654P.

12. Médicament comprenant des souches vivantes, des souches séchées ou des filtrats de culture de la souche de *Lactobacillus rhamnosus* PL60 KCCM-10654P.

13. Médicament pour utilisation dans la prévention ou le traitement des pathologies liées à l'obésité comprenant des souches vivantes, des souches séchées ou des filtrats de culture de la souche de *Lactobacillus rhamnosus* PL60 KCCM-10654P.

14. Médicament pour utilisation dans la prévention et le traitement des troubles liés à l'obésité selon la revendication 13, où des personnes saines ayant un poids moyen de 60 kg prennent la souche de *Lactobacillus rhamnosus* PL60 KCCM-10654P en une quantité de 1x10⁷ à 1x10¹¹ CFU par dose 1 à 2 fois par jour.
